# EUROPEAN PATENT APPLICATION

(11) **EP 1 900 332 A1**
(43) Date of publication of application: **19.03.2008**
(21) Application number: 06780640.6
(22) Date of filing: 23.06.2006
(51) Int. Cl.: A61B 17/32, A61B 18/12

(54) **ENDOSCOPE DEVICE**

(30) Priority: 30.06.2005 JP 2005191475
(71) Applicant: OLYMPUS MEDICAL SYSTEMS CORP., Shibuya-ku Tokyo 151-0072 (JP)
(72) Inventor: SUZUKI, Keita, 1900013 (JP)
(74) Representative: von Hellfeld, Axel
(86) International application number: PCT/JP2006/312603
(87) International publication number: WO 2007/004441

(57) **Abstract**

Incision forceps that are used by being passed through a forceps channel of an endoscope, comprising: a flexible tube that is inserted in a body cavity via the forceps channel; a long guide member that is disposed at a position projected from the distal end of the flexible tube; a forceps piece that is provided at the distal end and is provided to be capable of reciprocal movement between a near position that approaches the peripheral surface of the guide member and is projected from the distal end along the peripheral surface, and a far position F that is separated in a direction that intersects the axial line direction of the flexible tube with respect to the peripheral surface; **characterized by** the guide member being arranged on the axial line of the flexible tube.

## Description

### TECHNICAL FIELD

The present invention relates to an incision forceps used with an endoscope.
Priority is claimed on Japanese Patent Application No. 2005-191475, filed June 30, 2005, the content of which is incorporated herein by reference.

### BACKGROUND ART

In recent years, there have been increasing instances of performing various procedures using endoscopes when treating disorders in the digestive tract system and pancreaticobiliary system. In the treatment of the pancreaticobiliary system using an endoscope, a catheter is inserted from the duodenal papilla, and in addition to diagnostic treatments that image the bile duct and the pancreatic duct, there are also therapeutic measures which recover gallstones and the like that exist in the bile duct with a balloon or grasping device.

In the case of performing these treatments, since the opening of the duodenal papilla is narrow, difficulties arise when inserting various treatment devices in the bile duct. Therefore, in order to widen the opening of the duodenal papilla, a duodenal papilla incision operation is performed that makes an incision in the duodenal papilla by a papillotomy knife, in which an arched high-frequency electrode is provided at the distal end of the flexible tube. At this time, in order to cut the duodenal papilla in the optimal direction, using a flexible tube, it is necessary to rotate an RF electrode around the axial line of a flexible tube, and align this RF electrode at a suitable rotation position.

However, since it is not possible to support the distal end of the papillotomy knife in a body cavity, considerable skill is required to perform rapid and appropriate treatment at a target site in a stable manner without the distal end shaking during an operation. Also, if high-frequency forceps that enable an incision to be made while holding the target site with a pair of forceps (for example, refer to Patent Document 1) are used instead of a papillotomy knife, although the above stability is obtained, it is not easy to insert one of the forceps into the inner portion of a bile duct or the like. Even if it is possible to insert one forceps piece of the forceps, when a flexible tube is rotated for alignment in that state, the other forcep part of the forceps rotates with respect to the rotational axis of the flexible tube in the state of the one forcep part being pressed by the insertion portion, resulting in the rotational axis deviating, and so proper alignment of the forceps becomes difficult.
Patent Document 1: Japanese Unexamined Patent Application, first publication No. H05-253241

### DISCLOSURE OF INVENTION

### PROBLEM TO BE SOLVED BY THE INVENTION

The present invention was achieved in view of the above circumstances, and has as its object to provide incision forceps that can readily grasp or make an incision in a target site in a stable manner and achieve a shortening of the procedure time.

### MEANS FOR SOLVING THE PROBLEM

The present invention is an incision forceps that is used by being passed through a forceps channel of an endoscope, consisting of: a flexible tube that is inserted in a body cavity via the forceps channel; a long guide member that is disposed at a position projected from the distal end of the flexible tube; a forceps piece that is provided at the distal end and is provided to be capable of reciprocal movement between a near position that approaches the peripheral surface of the guide member and is projected from the distal end along the peripheral surface, and a far position that is separated in a direction that intersects the axial line direction of the flexible tube with respect to the peripheral surface; characterized by the guide member being arranged on the axial line of the flexible tube.

In this incision forceps, the guide member is inserted into the opening portion of the duodenal papilla, and in this insertion state, the flexible tube is made to rotate about the axial line. Then, the forceps piece rotates about the axial line of the flexible tube.
At this time, since the guide member is arranged on the axial line of the flexible tube, the forceps piece rotates about the axial line of the guide member and the flexible tube. When the forceps piece matches a predetermined rotation position, by arranging the forceps piece at the near position, the duodenal papilla is grasped by the guide member and the forceps piece. That is, the guide member functions as a piece of the forceps piece. In the state of being grasped thus, an incision is made in the duodenal papilla.
Thereby, not only is it possible to easily insert the guide member into the opening portion of the duodenal papilla as a piece of the forceps piece, but it is possible to easily rotate the forceps piece without moving the rotation axis when the flexible tube is rotated in the inserted state.

Also, the incision forceps in accordance with the present invention is the incision forceps in accordance with claim 1, characterized by a lumen that extends in the axial line direction being formed in the flexible tube, the guide member being formed in a cylindrical shape, and a tubular hole of the guide member and the lumen being connected.
In this incision forceps, in the state of the guide member being inserted into the opening portion of the duodenal papilla, when a contrast medium is injected for X-ray imaging for example from the rear end of the lumen, the contrast agent passes through the lumen and the tubular hole of the guide member, and flows out from the distal end of the guide member. That is, the lumen and the guide member function as imaging tubes. Then, after the X-ray imaging, the duodenal papilla is grasped and an incision is made.
Here, the treatment is conventionally performed as follows. First, the imaging tube is inserted in the duodenal papilla and the contrast medium is flowed. After the X-ray imaging, a guide wire is fed into the imaging tube, and then with that guide wire placed as is, the imaging tube is removed. Moreover, when a treatment tool for incision is fed along the guide wire that is placed, an incision in the duodenal papilla is performed with that treatment tool.

In the present invention, since the lumen and the guide member function as an imaging tube, it is possible to eliminate the insertion of a guide wire for making an incision in the duodenal papilla, and it is possible to perform treatment quickly and easily.
In order to quicken the treatment, it is conceivable to arrange the imaging lumen and the flexible tube of the incision forceps in parallel, and then feed the imaging lumen and the flexible tube simultaneously. However, arranging in parallel as above increases the overall diameter. In the present invention, since it is possible to make the lumen and the guide member function as an imaging tube, it is possible to maintain an overall narrow diameter, and it is possible to improve the insertion to and extraction from the forceps channel of the endoscope.

Also, the incision forceps in accordance with the present invention is the incision forceps in accordance with claim 1, characterized by a lumen that extends in the axial line direction being formed in the flexible tube, the guide member being formed in a cylindrical shape, and the guide member being capable of insertion to and extraction from the lumen via a distal end opening portion of the lumen from the rear end of the guide member, and the rear end of the guide member passing through the lumen and being capable of being advanced and retracted from a rear end opening portion of the lumen.

In this incision forceps, in the state of the guide member being inserted into the opening portion of the duodenal papilla, a contrast medium is injected from the rear end of the guide member. After X-ray imaging, the rear end of the guide member is inserted in the lumen via the distal end opening portion of the lumen, and the flexible tube is fed along the guide member. Then, the duodenal papilla is grasped and an incision is made therein by the forceps piece and the guide member.
Thereby, not only is it possible to exhibit the same effect as the invention in accordance with claim 2, but it is possible to utilize an imaging tube as a guide member, and therefore there is no need to newly provide a special-purpose guide tube, it is possible to enhance the general-purpose properties and convenience.

Also, the incision forceps in accordance with the present invention is the incision forceps in accordance with any one of claims 1 to 3, characterized by the flexible tube being provided with a coil tube that is formed in a coil shape and an outer tube that covers this coil tube on the same axis; and the coil tube and the outer tube being mutually rotatable about the axis of the flexible tube.

In this incision forceps, in the state of the guide member being inserted into the opening portion of the duodenal papilla, rotation of the flexible tube causes the coil tube to rotate with respect to the outer tube.
Thereby, it is possible to easily rotate the forceps piece via the coil tube even when friction is present between the outer tube and the outside portion.

Also, the incision forceps in accordance with the present invention is the incision forceps in accordance with any one of claims 1 to 3, characterized by the guide member being capable of rotating about the axial line of the flexible tube.

In this incision forceps, in the state of the guide member being inserted into the opening portion of the duodenal papilla, rotation of the flexible tube causes the flexible tube to rotate with respect to the guide member.
Thereby, it is possible to easily rotate the flexible tube and the forceps piece even when friction is present between the guide member and the outside portion.

Also, the incision forceps in accordance with the present invention is the incision forceps in accordance with any one of claims 1 to 5, characterized by the guide member being detachably provided at the distal end of the flexible tube.

In this incision forceps, the guide member is detachably attached to the distal end of the flexible tube. Here, the length and shape of the required guide member differs depending on the size and state of the duodenal papilla.
In the present invention, since the guide member is detachable, it is possible to easily attach the optimal guide member depending on the situation, and it is possible to perform the optimal procedure in a short time.

Also, the incision forceps in accordance with the present invention is the incision forceps in accordance with any one of claims 1 to 6, characterized by a reinforcing member being provided at a flexed portion of the flexible tube when raised by a forceps elevator base that is provided in the forceps channel as a result of the flexible tube being passed through the forceps channel of the endoscope.

In this incision forceps, when the flexible tube is inserted in the forceps channel and the forceps elevator base is operated, the flexible tube flexes and rises by the forceps elevator base. At this time, since the reinforcing member is provided at the flexed portion, it is possible to improve the durability of the flexible tube.

Also, the incision forceps in accordance with the present invention is the incision forceps in accordance with any one of claims 1 to 7, characterized by a distal end cover with insulating properties being provided at the distal end of the flexible tube.

In this incision forceps, when a high-frequency current is flowed to the forceps piece in the state of the duodenal papilla being grasped, the duodenal papilla is cauterized, and an incision is thereby performed. At this time, since the distal end cover with insulating properties is provided at the distal end of the flexible tube, it is possible to prevent high-frequency current from flowing to living body tissue via the distal end cover, and possible to efficiently supply high-frequency current to the forceps piece.

### EFFECT OF THE INVENTION

In accordance with the present invention, not only is it possible to easily insert the guide member into the opening portion of the duodenal papilla, but it is possible to easily rotate the guide member and the forceps piece without moving the rotation axis when the flexible tube is rotated in the inserted state. Therefore, it is possible to readily grasp or make an incision in a target site in a stable manner and achieve a shortening of the procedure time.

### BRIEF DESCRIPTION OF THE DRAWINGS

FIG 1 is an explanatory drawing showing the first embodiment of the incision forceps in accordance with the present invention, and showing the aspect of performing various treatments by use with an endoscope.
FIG 2 is a sectional side view showing an enlargement of the sheath portion of FIG 1.
FIG 3 is an explanatory drawing showing in magnification the operation portion of the incision forceps and the forceps attachment portion of FIG. 1.
FIG 4 is a sectional side view showing in magnification the distal end portion of the incision forceps of FIG 1.
FIG 5 is a sectional view along line A-A of FIG 4.
FIG 6 is an explanatory drawing showing the aspect of the forceps piece of FIG. 1 being arranged in the near position and grasping the duodenal papilla.
FIG 7 is a explanatory drawing showing the main portions of the second embodiment of the incision forceps in accordance with the present invention, showing the aspect of the rear end of the guide tube being inserted in the lumen 47 via the distal end opening portion.
FIG 8 is an explanatory drawing showing the aspect of the rear end of the guide tube of FIG 7 projecting out via the injection opening portion.
FIG 9 is an explanatory drawing showing the aspect of using the guide tube and incision forceps of FIG 7 to perform an incision, showing the aspect of the guide tube being inserted in the papilla opening portion.
FIG 10 is an explanatory drawing showing the aspect of using the guide tube and incision forceps of FIG 7 to perform an incision, showing the aspect of feeding the incision forceps via the guide tube.
FIG 11 is an explanatory drawing showing the aspect of using the guide tube and incision forceps of FIG 7 to perform an incision, showing the aspect of grasping the duodenal papilla.
FIG 12 is a sectional side view showing the main portions of the third embodiment of the incision forceps in accordance with the present invention.
FIG. 13 is a side view showing the main portions of the fourth embodiment of the incision forceps in accordance with the present invention.
FIG. 14 is a side view showing the main portions of the fifth embodiment of the incision forceps in accordance with the present invention.
FIG. 15 is a side view showing a modification example of the reinforcing tube of FIG 14.

### BRIEF DESCRIPTION OF THE REFERENCE SYMBOLS

1 incision forceps
2 endoscope
11 forceps channel
15 forceps elevator base
16 sheath portion (flexible tube)
17 coil tube
20 outer tube
32 distal end cover
35 forceps piece
45, 45a, 45b, 45b, 45d, 45e, 45f guide tube (guide member)
47 lumen
50 tubular hole
56 injection opening hole (rear end opening portion)
65 distal end opening portion
76 reinforcing tube (reinforcing member) axial line N near position, F far position of L flexible tube

### BEST MODE FOR CARRYING OUT THE INVENTION

Next, the embodiments of the present invention shall be described based on the drawings. Note that in the following explanations of each embodiment, the same reference numerals shall be assigned to identical portions, with overlapping explanations being omitted.

### (Embodiment 1)

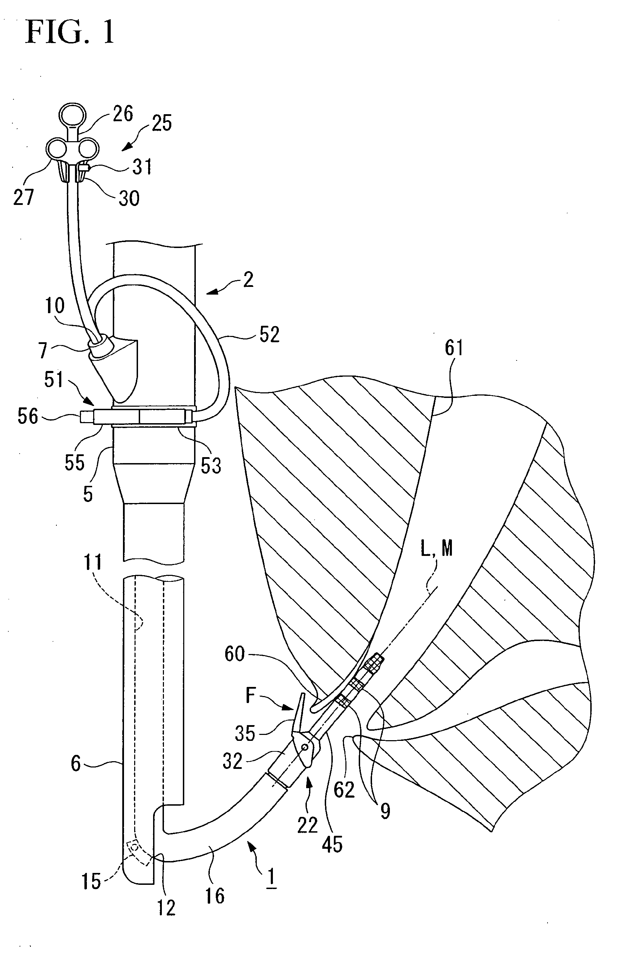

Incision forceps in a first embodiment of the present invention are described below with reference to the drawings.
As shown in FIG. 1, an incision forceps 1 in the present invention performs treatment by being used with an endoscope 2.
Therefore, the endoscope 2 that is used with the incision forceps 1 shall first be described.
The main constituent elements of the endoscope 2 are an endoscope operation portion 5 that the operator holds in his hand and performs various operations, and an endoscope insertion portion 6 that is inserted into a body cavity. That is, the endoscope 2 is constituted by the endoscope operation portion 5 being attached to the proximal side end of the endoscope insertion portion 6 that is hollow and elongated.

A forceps plug 7 for inserting various tools is provided in the endoscope operation portion 5. A forceps plug opening portion 10 that serves as the insertion opening for various treatment tools is formed in the forceps plug 7.
A tubular forceps channel 11 that is an insertion passage for various treatment tools is formed in the endoscope insertion portion 6. Also, a channel exit opening portion 12 is formed at the distal end of the endoscope insertion portion 6. One end of the forceps channel 11 serves as the forceps plug opening portion 10, and the other end serves as the channel exit opening portion 12. Also, a forceps elevator base 15 for activating various treatment tools is provided in the vicinity of the channel exit opening portion 12, which is the distal end portion of the forceps channel 11..
With such a constitution, when various kinds of treatment tools are inserted and led from the forceps plug opening portion 10, the treatment tool passes through the forceps channel 11 to protrude outward from the channel exit opening portion 12.

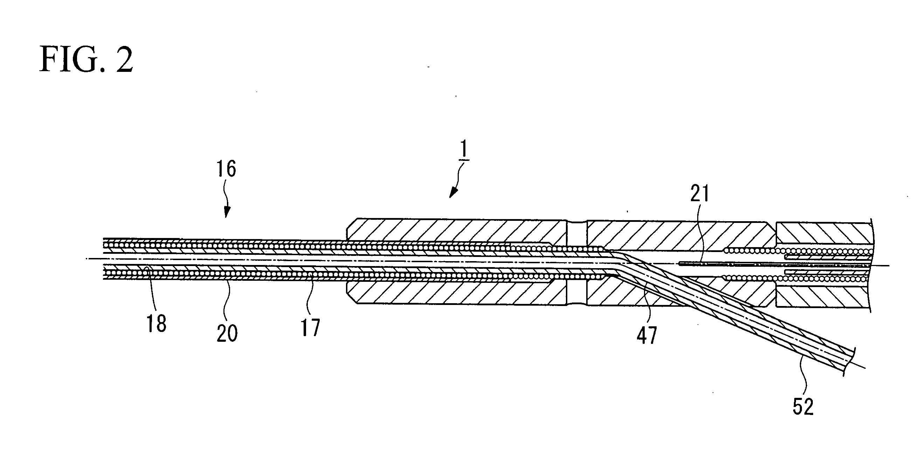

Next, the incision forceps 1 in accordance with the present invention shall be described.
As shown in FIG 1 to FIG 3, the incision forceps 1 is provided with an insulating sheath portion (flexible tube) 16 that has flexibility and extends in a cylindrical shape. The sheath portion 16, as shown in FIG 2, is provided with a coil tube 17 that is formed by a wound wire, and an outer tube 20 is provided on the same axis so as to cover the peripheral surface thereof. A through-hole 18 that extends in the length direction is formed inside of the sheath portion 16, and an operational wire 21 is passed through this through-hole 18. This operational wire 21 is made from a metal wire such as stainless steel or steel, and can be made to advance and retract with respect to the sheath portion 16. A treatment portion 22 to be described later is provided at the distal end of the operational wire 21, as shown in FIG 1.

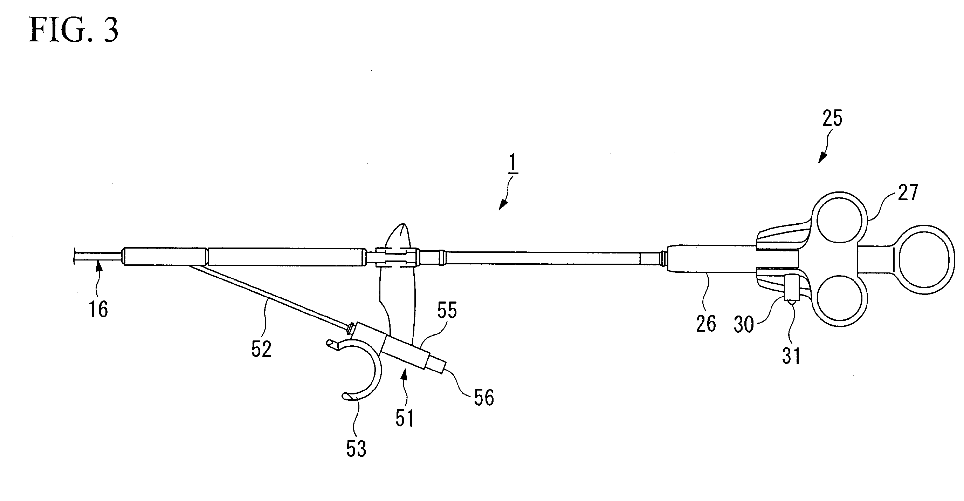

Also, a distal end cover 32 to be described later is provided at the distal end of the sheath portion 16. An operation portion 25 for performing various operations is connected to the rear end of the sheath portion 16.
As shown in FIG 3, the operation portion 25 is provided with an operation body portion 26 that extends in the axial line direction of the sheath portion 16, and a slide portion 27 that is supported so as to be capable of advancing and retracting with respect to the operation body portion 26, through which this operation body portion 26 is inserted. The rear end of the operational wire 21 is attached to the slide portion 27.
And by making the slide portion 27 advance and retract with respect to the operation body portion 26, the operational wire 21 advances and retracts with respect to the sheath portion 16. Also, a connection portion 30 for connection to a high-frequency power supply not illustrated is provided in the slider portion 27. The connection portion 30 is provided with an electrode terminal 31 that extends in a column shape, and this electrode terminal 31 is electrically connected to the operational wire 21.
With such a constitution, by connecting a cable not illustrated that extends from the high-frequency power supply to the connection portion 30 and driving the high-frequency power supply, a high-frequency current flows to the operational wire 21 via the cable and the electrode terminal 31.

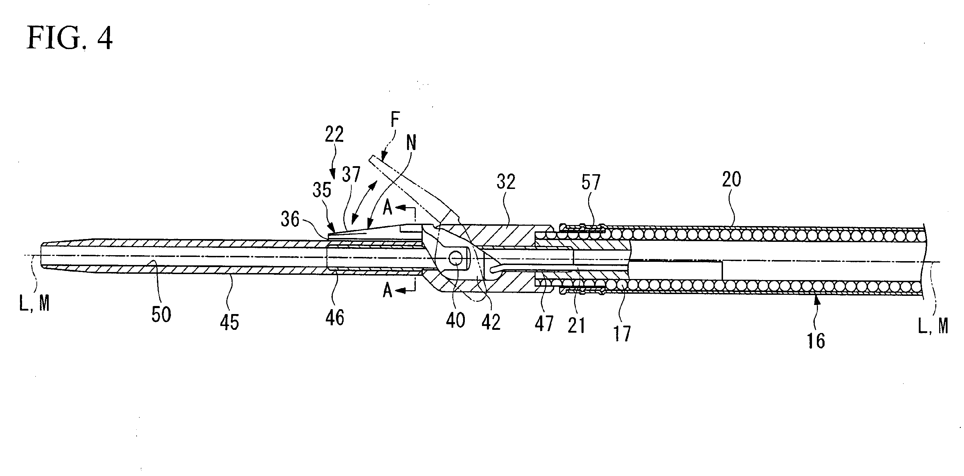

Moreover, as shown in FIG. 4, the incision forceps 1 in the present embodiment is provided with a guide tube (guide member) 45 that has pliability and extends lengthwise. This guide tube 45 consists of an insulation material, and is formed in a tubular shape. Also, the guide tube 45 is attached to the distal end of a sheath portion 16 via an attachment cylinder portion 46 that projects from the distal end of the sheath portion 16 along the axial line L thereof. That is, the guide tube 45 is disposed at a position that projects from the distal end of the sheath portion 16. Moreover, the guide tube 45 is disposed on the axial line L of the sheath portion 16, and the axial line M of the guide tube 45 and the axial line L of the sheath portion 16 agree. Also, in order to facilitate insertion of the distal end portion of the guide tube 45 into a papilla opening portion and the like, it is formed so as to gradually decrease in diameter toward the distal end. As shown in FIG 1, in order to know the insertion distance of the guide tube 45, markings 9 are provided at a predetermined spacing on the peripheral surface of the guide tube 45.

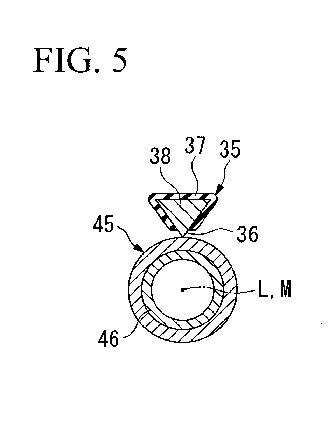

Also, the treatment portion 22 is provided with a single forceps piece 35 that is attached to the distal end of the sheath portion 16 via a coupling member 42. As shown in FIG 5, the forceps piece 35 is provided with a forceps body 38 that has electrical conductivity and is formed so that the transverse section thereof has a triangular shape. The forceps body 38 is electrically connected to the operational wire 21 via the coupling member 42. An insulating coating film 37 that covers the entire forceps body 38 except for one vertex portion that is disposed on the guide tube 45 side among the three vertex portions is provided on the peripheral surface of the forceps body 38. The insulating coating film 37 consists of an insulating resin such as polytetrafluoroethylene (PTFE), ethylene tetrafluoroethylene copolymer (ETFE), polyetheretherketone (PEEK), and an insulating member such as ceramics including alumina and zirconia, diamond like carbon (DLC), and diamond like nanocomposite (DLN). The vertex portion that is exposed to the outside is a treatment electrode portion 36 that incises a portion to be incised by high-frequency current.
Also, the coupling member 42 is coupled to the distal end of the operational wire 21. Moreover, the coupling member 42 is attached to the distal end cover 32 by a mounting pin 40 that is oriented in a direction that is perpendicular to the axial line L of the sheath portion 16. Thereby, advancing and retracting the operational wire 21 causes the coupling member 42 to rotate in forward and reverse directions centered on the axial line of the mounting pin 40.

With such a constitution, by advancing and retracting the operational wire 21, the forceps piece 35 opens and closes with respect to the guide tube 45 via the coupling member 42. That is, by advancing and retracting the operational wire 21, the forceps piece 35, via the coupling member 42, is disposed at a near position N that is a position near the peripheral surface of the guide tube 45 and projects from the distal end of the sheath portion 16 along the peripheral surface thereof (refer to FIG 4). On the other hand, advancing the operational wire 21 causes the forceps piece 35 to be disposed at a far position F that is separated in a direction that is perpendicular with the axial line L of the sheath portion 16 with respect to the peripheral surface of the guide tube 45 via the coupling member 42.
Moreover, the projecting dimension of the forceps piece 35 from the distal end of the sheath portion 16 when disposed in the near position N is set so as to be shorter than the projecting dimension of the guide tube 45.

Also, a tubular lumen 47 is provided in the through-hole 18 of the sheath portion 16 parallel with the operational wire 21. The distal end of this lumen 47 is connected with a tubular hole 50 of the guide tube 45 via the attachment cylinder portion 46. On the other hand, the rear end of the lumen 47, as show in FIG. 2 and FIG. 3, is the base end side of the sheath portion 16, and extends until a forceps attachment portion 51 that is provided in the vicinity of the operation portion 25. That is, the lumen 47 is extended until a cylindrical main cylinder portion 55 that is attached via a coupling pipe 52. Thereby, an injection opening portion 56 of the main cylinder portion 55 is connected until the distal end of the guide tube 45 through the lumen 47. An attachment hook 53 for attachment to an endoscope insertion portion 6 is attached to the forceps attachment portion 51.

Moreover, as shown in FIG. 4, an annular connection portion 57 is provided at the distal end of the outer tube 20, and so the outer tube 20 and the coil tube 17 can be mutually rotated around the axial line L via the annular connection portion 57. The distal end cover 32 is provided at the distal end of the coil tube 17, and so by rotating the coil tube 17, the distal end cover 32, the forceps piece 35, and the guide tube 45 rotate about an axial lines L, M with respect to the outer tube 20.
Also, the distal end cover 32 consists of an insulating member. As the insulating member, ceramics including such as alumina and zirconia, or fluororesins such as PTFE, ETFE, and resins such as PEEK are used.

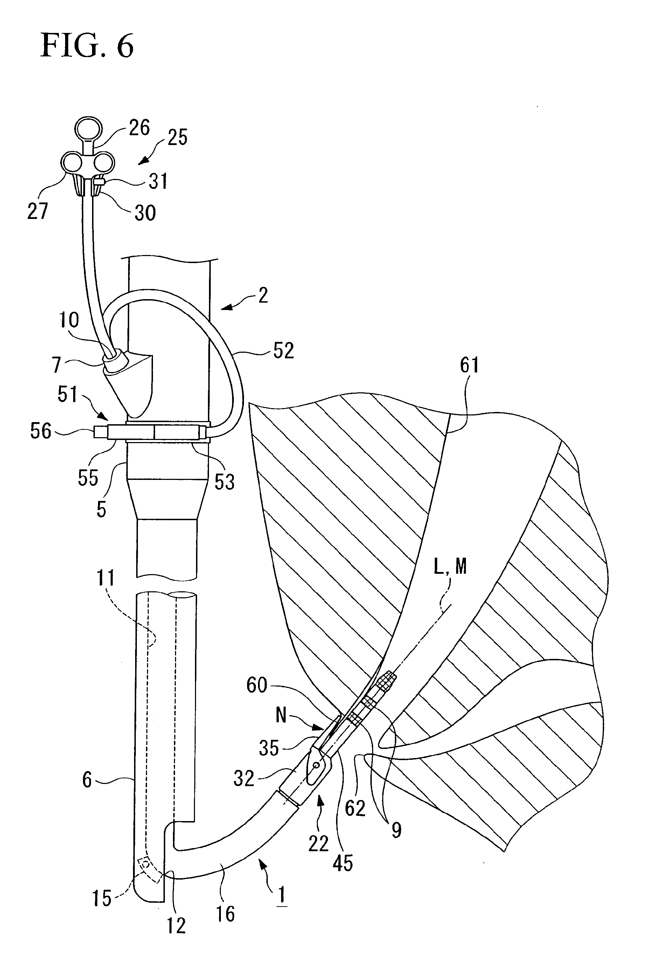

Next, the action of the incision forceps 1 in the present embodiment constituted in this manner shall be described using FIG 1 and FIG. 6.
Note that FIG 1 and FIG 6 describe a procedure of inserting the endoscope insertion portion 6 in the duodenum, and inserting the incision forceps 1 from the papilla opening portion 62 of the duodenal papilla 60 to the bile duct 61.
First, the endoscope insertion portion 6 is inserted in the subject, and fed until the vicinity of the duodenal papilla 60. Then, by inserting the incision forceps 1 from the forceps plug opening portion 10, the guide tube 45 etc. is projected outward from the channel exit opening portion 12. At this time, depending on the condition, the forceps elevator base 15 is activated, and the sheath portion 16 is elevated. Then, the sheath portion 16 is fed, and as shown in FIG. 1, in the state of the forceps piece 35 being disposed at the far position F, the guide tube 45 is inserted into the papilla opening portion 62.

In the state of the guide tube 45 being inserted, a contrast medium is injected from the injection opening portion 56 of the forceps attachment portion 51 that is disposed on the proximal side. Then, the contrast medium flows from the distal end of the guide tube 45 via the lumen 47 and the tubular hole 50 of the guide tube 45. At this time, the lumen 47 and the guide tube 45 function as an imaging tube. Then, X-ray imaging is performed in the state of the contrast medium being filled in the bile duct 61. It is determined based on this X-ray imaging whether or not treatment in the bile duct 61 is required. Here, in the case of treatment being determined to be necessary and the papilla opening portion 62 being narrow, an incision in the papilla opening portion 62 is performed. That is, the incision forceps 1 is connected to a high-frequency power supply, and in the state of the guide tube 45 being inserted, the coil tube 17 is rotated around the axial line L via the operation portion 25 that is disposed on the proximal side. Then, with the outer tube 20 remaining stationary, only the coil tube 17 is rotated. The rotation is transmitted to the distal end of the coil tube 17, and the guide tube 45 rotates about the axial lines L and M. Simultaneously, the forceps piece 35 also rotates about the axial lines L and M.

Then, after the forceps piece 35 is disposed at a predetermined rotation position, the forceps piece 35 is closed with respect to the guide tube 45. That is, as shown in FIG 6, the forceps piece 35 is put in the near position N. Thereby, the duodenal papilla 60 is grasped by the forceps piece 35 and the guide tube 45. At this time, the guide tube 45 functions as one forceps piece. Then, by driving the high-frequency power supply, high-frequency current is flowed to the treatment electrode portion 36 via the operation wire 21 and the like. At this time, since the distal end cover 32 consists of an insulating member, the high-frequency current that flows from the operation wire 21 does not flow through the distal end cover 32. Then, the duodenal papilla 60 is cauterized by the high-frequency current from the treatment electrode portion 36, and an incision in the duodenal papilla 60 is performed. Then, after an incision is made in the duodenal papilla 60, the incision forceps 1 is drawn out with the endoscope insertion portion 6 being held in place, and moreover various treatment tools are inserted via the endoscope insertion portion 6, with a predetermined treatment being performed on the bile duct 61.

Thereby, in accordance with the incision forceps 1 in the present embodiment, since the projecting dimension of the guide tube 45 is longer than the projecting dimension of the forceps piece 35 and the distal end becomes narrower in diameter, the guide tube 45 can be readily inserted in the papilla opening portion 62. Moreover, since the guide tube 45 is disposed on the axial line L of the sheath portion 16, and the axial line L and the axial line M of the guide tube 45 agree, in the state of the guide tube 45 being inserted in the papilla opening portion 62, when the sheath portion 16 is rotated, it is possible to easily rotate the forceps piece 35 without shifting the rotation axis. For that reason, it is possible to easily grasp or make an incision in the duodenal papilla 60 in a stable manner, and possible to achieve a shortening of the procedure time.

Also, since the lumen 47 and the guide tube 45 function as imaging tubes, it is possible to eliminate the insertion of a guide wire for making an incision in the duodenal papilla 60, and possible to perform treatment quickly and easily. Moreover, since it is not necessary to insert an imaging lumen and the sheath portion 16 in parallel, it is possible to maintain the overall narrow diameter, and possible to improve the insertion-and-extraction characteristic to/from the forceps channel 11.
Also, since the outer tube 20 and the coil tube 17 can mutually rotate, it is possible to readily rotate the forceps piece 35 via the coil tube 17 even when friction is present between the outer tube 20 and the outside portion.
Moreover, since the distal end cover 32 is formed from an insulating member, it is possible to prevent high-frequency current from flowing to living body tissue via the distal end cover 32, and possible to efficiently supply high-frequency current to the treatment electrode portion 36.

Note that, in the present embodiment, the guide tube 45 is formed in a tubular shape, but is not limited thereto, and the shape can be appropriately altered. For example, it may be a simple bar shape. However, by making it a tube shape and utilizing the tubular hole, it is possible to have it function as an imaging lumen as above.
Also, the coil tube 17 and the outer tube 20 were made to be able to mutually rotate, but are not limited thereto, and both may be fixed. However, making them capable of rotation can further improve the rotation operatability, as above.
Also, the distal end cover 32 was made to consist of an insulating member, but is not limited thereto, and the material can be suitably modified. For example, it may be stainless steel or the like, with an insulation coating applied to the surface of the stainless steel.

### (Embodiment 2)

Next, the second embodiment of the present invention shall be described.

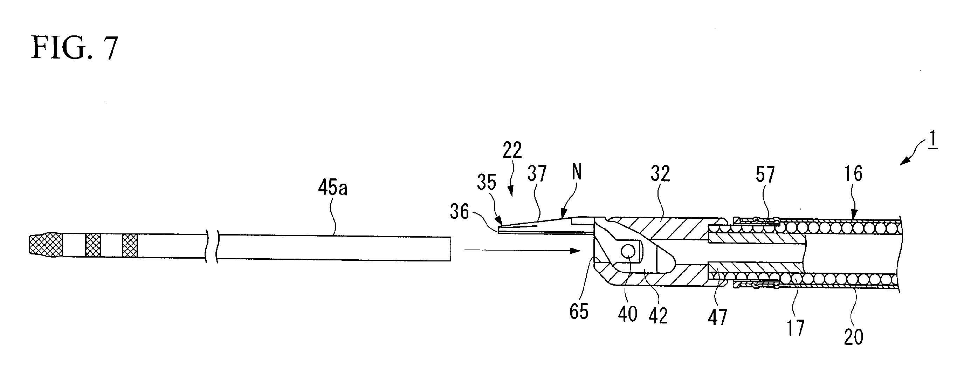

FIG. 7 to FIG. 11 show the second embodiment of the present invention.
In FIG. 7 to FIG. 11, portions identical to constituent elements disclosed in FIG. 1 to FIG 6 shall be assigned the same reference numerals and explanations thereof shall be omitted here.
The basic constitution of this embodiment is identical to the first embodiment, and so only the points of difference shall be given here.

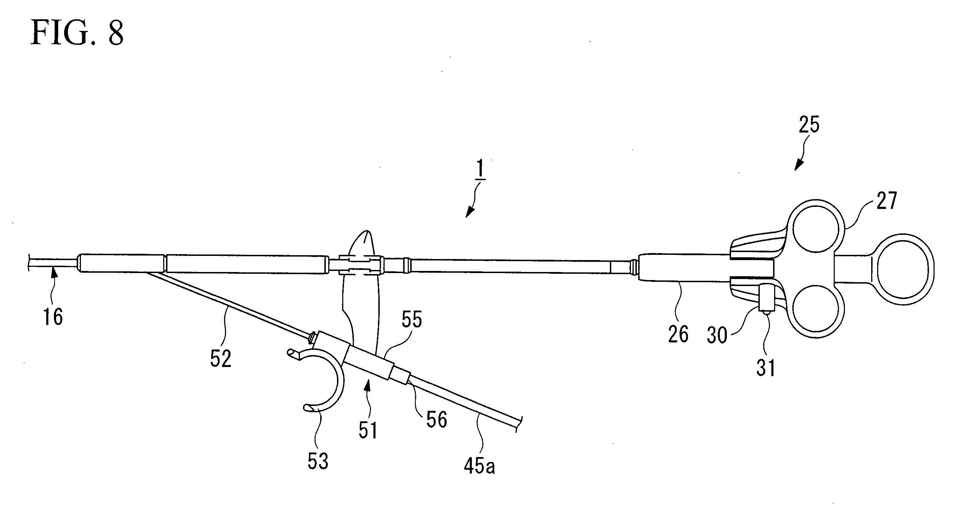

In the present embodiment, as shown in FIG. 7, an opening portion is formed at the distal end of the distal end cover 32, and this opening portion is opened wide. That is, the opening portion of the distal end cover 32 serves as a distal end opening portion 65 of the lumen 47. On the other hand, as shown in FIG 8, the injection opening portion 56 of the forceps attachment portion 51 serves as a rear end opening portion.
Also, a guide tube 45a is formed to be longer than the guide tube 45 in the first embodiment. The length dimension thereof is for example set to be approximately the same as a conventional imaging tube. Then, as shown in FIG 7, the rear end of the guide tube 45a can be inserted and detached in the lumen 47 via the distal end opening portion 65. That is, when the rear end of the guide tube 45a is inserted in the lumen 47, the guide tube 45a is disposed at a position that projects from the distal end of the sheath portion 16. Moreover, when the sheath portion 16 is made to advance with respect to the guide tube 45a by inserting the rear end of the guide tube 45a in the lumen 47, as shown in FIG 8, the rear end of the guide tube 45a comes to project outward from the injection opening portion 56 by passing through the lumen 47. Then, it is possible to make the guide tube 45a advance and retract with respect to the lumen 47 via the guide tube 45a projected from the injection opening portion 56.

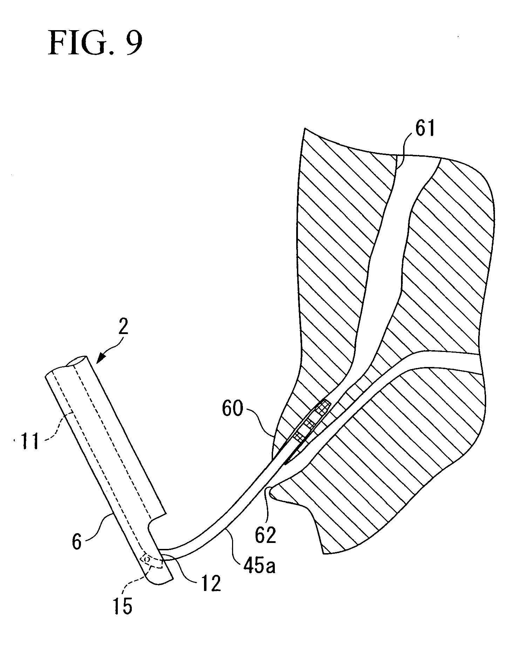

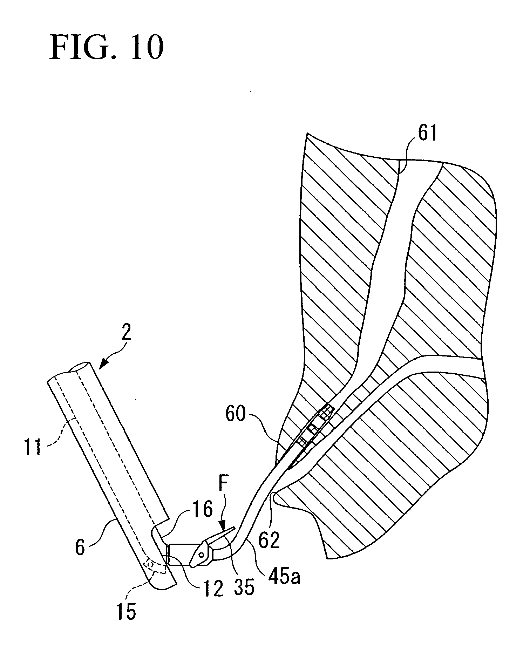

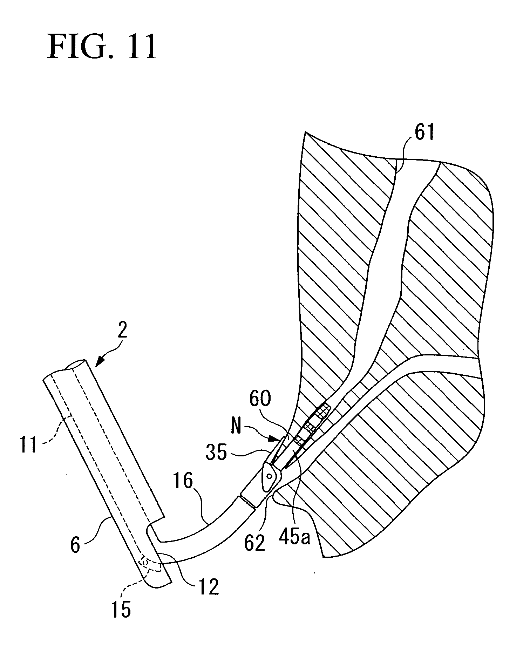

With this constitution, to make an incision in the duodenal papilla 60, as shown in FIG 9, only the guide tube 45a is inserted in the papilla opening portion 62 via the endoscope insertion portion 6. Then, similarly to above, a contrast medium is injected and X-ray imaging is performed. Here, in the case of any treatment being required on the bile duct 61 or the like, by inserting the rear end of the guide tube 45a that extends to the proximal side of the endoscope insertion portion 6 in the lumen 47 via the distal end opening portion 65, as shown in FIG 10, the sheath portion 16 is made to advance with respect to the guide tube 45a. Then, the forceps piece 35 is fed until the grasping surface of the forceps piece 35 is disposed at a position facing the duodenal papilla 60. Moreover, by having the forceps piece 35 match a predetermined rotation position, as shown in FIG 11, the forceps piece 35 is arranged at the near position N. Thereby, the duodenal papilla 60 is grasped. Then, an incision is performed by flowing high-frequency current, and by extracting the incision forceps 1 from the endoscope insertion portion 6, various treatments on the bile duct 61 are performed by various treatment tools.

Thereby, not only is it possible to exhibit the same effect as the incision forceps 1 in accordance with the first embodiment, but it is possible to utilize a conventional imaging tube as the guide tube 45a. For that reason, since there is no need to newly prepare a special-purpose guide tube, it is possible to enhance the general-purpose properties and convenience.

### (Embodiment 3)

Next, the third embodiment of the present invention shall be described.

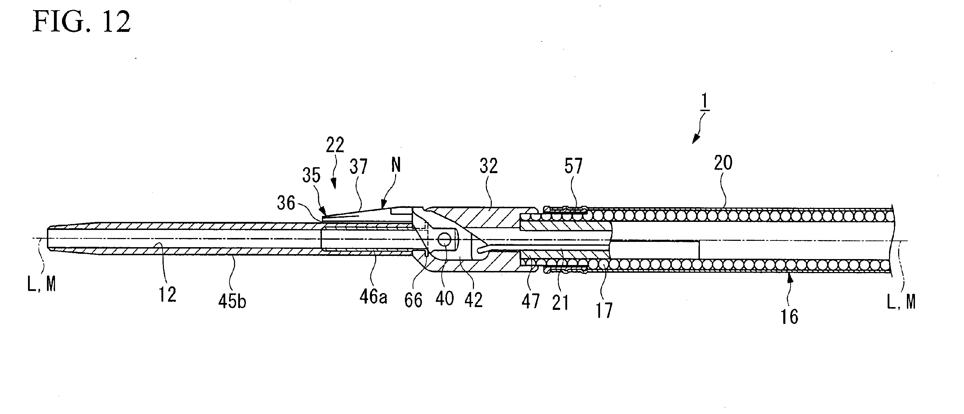

FIG 12 shows the third embodiment of the present invention.
In the present embodiment, a guide tube 45b is attached at the distal end of the distal end cover 32 to be rotatable about the axial lines L and M with respect to the sheath portion 16 and the forceps piece 35. That is, a flange portion 66 is provided at the base end portion of the attachment cylinder portion 46a, and this flange portion 66 functions as a retaining portion of the attachment cylinder portion 46a from the distal end cover 32. The attachment cylinder portion 46a is rotatable about the axial line L, and the guide tube 45b is attached to this attachment cylinder portion 46a.

With this kind of constitution, when the coil tube 17 is rotated in the state of the guide tube 45b being inserted in the papilla opening portion 62, the forceps piece 35 rotates about the axial lines L and M with the guide tube 45b remaining stationary.
Therefore, it is possible to easily rotate the forceps piece 35 even when friction is present between the guide tube 45b and the outside portion.

### (Embodiment 4)

Next, the fourth embodiment of the present invention shall be described.

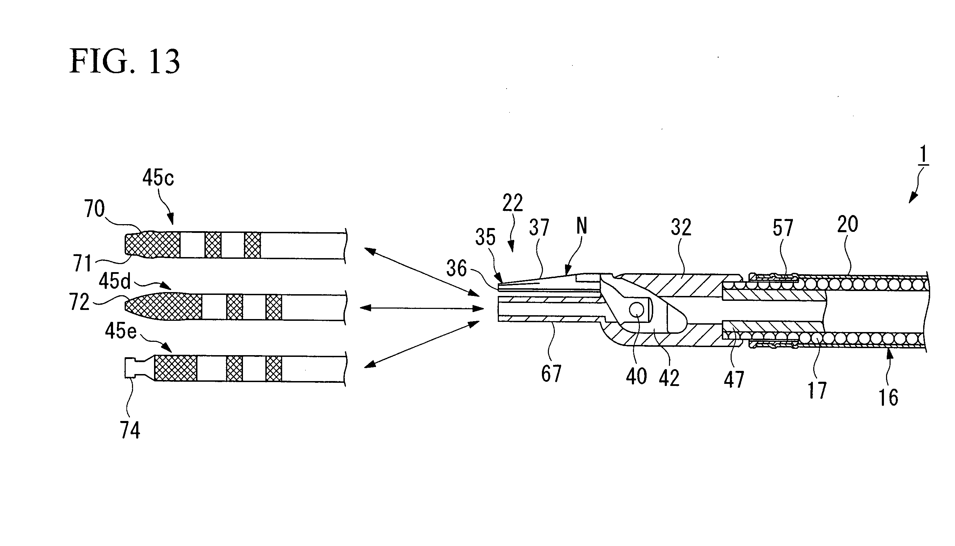

FIG 13 shows the fourth embodiment of the present invention.
In the present embodiment, at the distal end of the distal end cover 32, a cylindrical projection portion 67 that projects from the distal end thereof is provided. Guide tubes 45c, 45d, 45e alternatively fit on this cylindrical projection portion 67. By fitting the guide tubes 45c, 45d, 45e on the cylindrical projection portion 67, the guide tubes 45c, 45d, 45e are attached to the distal end of the distal end cover 32 in selective and detachable manner. That is, the guide tubes 45c, 45d, 45e are prepared in a plurality of types, and these guide tubes 45c, 45d, 45e are attached in an exchangeable manner.

A uniform diameter insertion portion 71 that has the same diameter as the base end portion is provided at the distal end of the guide tube 45c via a distal end narrow diameter portion 70. This guide tube 45c is a standard type and a type with the highest versatility.
A sloping portion 72 in which the diameter gradually narrows is provided at the distal end of the guide tube 45d. This guide tube 45d is used in cases when the papilla opening portion 62 is small, leading to insertion difficulties.
A metal insertion portion 74, which is made of metal, is provided at the distal end of the guide tube 45e. This guide tube 45e is inserted in the papilla opening portion 62 via the metal insertion portion 74, being used in cases when insertion into the papilla opening portion 62 is difficult.

Since these guide tubes 45c, 45d, 45e are selectable and detachable, it is possible to easily attach the optimal guide tube 45c, 45d or 45e depending on the situation, and it is possible to perform the optimal procedure in a short time.
Note that the guide tubes 45c, 45d, 45e were given as the three types, but are not limited thereto, and the shape and number can be suitably modified.

### (Embodiment 5)

Next, the fifth embodiment of the present invention shall be described.

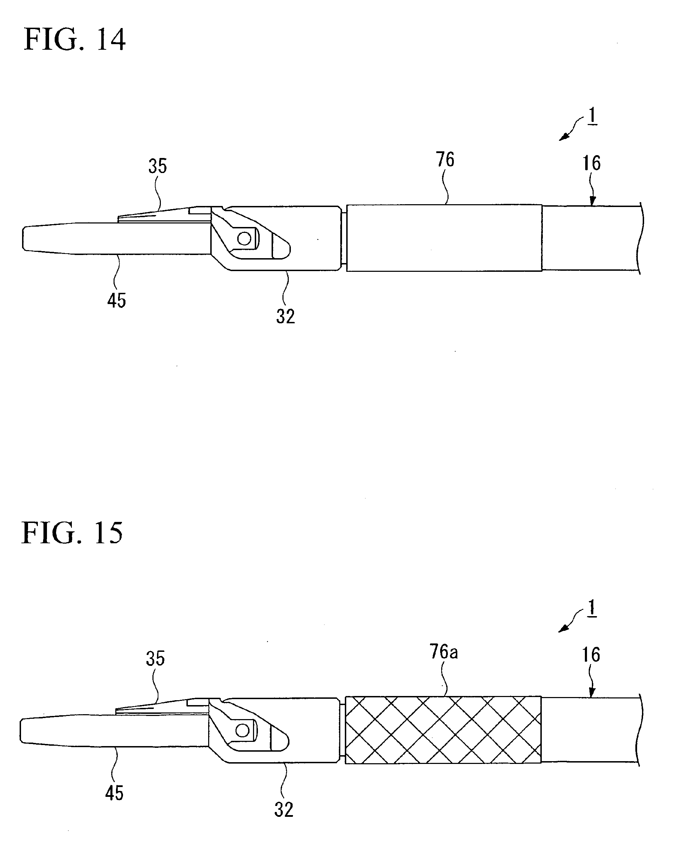

FIG 14 shows the fifth embodiment of the present invention.
In the present embodiment, a reinforcing tube (reinforcing member) 76 that is made of an elastic material is provided near the distal end portion (distal end cover 32) of the sheath portion 16.
Here, by activating the forceps elevator base 15 in the state of the incision forceps 1 being inserted in the forceps channel 11, the sheath portion 16 flexes and rises by the forceps elevator base 15. In the present embodiment, since the reinforcing tube 76 is provided at the flexed portion, when the sheath portion 16 is raised, that flexed portion is reinforced. For that reason, it is possible to improve the durability of the sheath portion 16.

Note that in the present embodiment, the reinforcing tube 76 is provided, but the shape and material, etc. thereof can be suitably modified. For example, as shown in FIG. 15, the reinforcing tube 76a made of woven metal wire may be provided.
Also, in the first through fifth embodiments, the shape of the transverse cross section of the forceps piece 35 was triangular, but is not limited thereto, and may be suitably modified.
Also, the shape and material, etc. of the guide tube 45 can be suitably modified.
Note that the technical scope of the present invention is not limited to the embodiments disclosed above, and various modifications can be made without departing from the spirit or scope of the present invention.

### INDUSTRIAL APPLICABILITY

The incision forceps in accordance with the present invention can be favorably used for medical applications.

## Claims

1. An incision forceps that is used by being passed through a forceps channel of an endoscope, comprising:
a flexible tube that is inserted in a body cavity via the forceps channel;
a long guide member that is disposed at a position projected from the distal end of the flexible tube;
a forceps piece that is provided at the distal end and is provided to be capable of reciprocal movement between a near position that approaches the peripheral surface of the guide member and is projected from the distal end along the peripheral surface, and a far position that is separated in a direction that intersects the axial line direction of the flexible tube with respect to the peripheral surface;
**characterized by** the guide member being arranged on the axial line of the flexible tube.

2. The incision forceps in accordance with claim 1, **characterized by**
a lumen that extends in the axial line direction being formed in the flexible tube,
the guide member being formed in a cylindrical shape, and
a tubular hole of the guide member and the lumen being connected.

3. The incision forceps in accordance with claim 1, **characterized by**
a lumen that extends in the axial line direction being formed in the flexible tube,
the guide member being formed in a cylindrical shape, and
the guide member being capable of insertion to and extraction from the lumen via a distal end opening portion of the lumen from the rear end of the guide member, and the rear end of the guide member passing through the lumen and being capable of being advanced and retracted from a rear end opening portion of the lumen.

4. The incision forceps in accordance with any one of claims 1 to 3, **characterized by**:
the flexible tube being provided with a coil tube that is formed in a coil shape and an outer tube that covers this coil tube on the same axis; and
the coil tube and the outer tube being mutually rotatable about the axial line of the flexible tube.

5. The incision forceps in accordance with any one of claims 1 to 4, **characterized by** the guide member being rotatable about the axial line of the flexible tube.

6. The incision forceps in accordance with any one of claims 1 to 5, **characterized by** the guide member being detachably provided at the distal end of the flexible tube.

7. The incision forceps in accordance with any one of claims 1 to 6, **characterized by** a reinforcing member being provided at a flexed portion of the flexible tube when raised by a forceps elevator base that is provided in the forceps channel, in which the flexible tube is passed through the forceps channel of the endoscope.

8. The incision forceps in accordance with any one of claims 1 to 7, **characterized by** a distal end cover with insulating properties being provided at the distal end of the flexible tube.
